(19) Europäisches Patentamt
European Patent Office
Office européen des brevets

(11) **EP 2 623 103 A1**

(12) **EUROPEAN PATENT APPLICATION**
published in accordance with Art. 153(4) EPC

(43) Date of publication:
07.08.2013 Bulletin 2013/32

(51) Int Cl.:
*A61K 31/445* (2006.01)  *A61K 9/70* (2006.01)
*A61K 47/14* (2006.01)  *A61K 47/38* (2006.01)
*A61P 25/28* (2006.01)

(21) Application number: 11829354.7

(22) Date of filing: 30.09.2011

(86) International application number:
PCT/JP2011/072579

(87) International publication number:
WO 2012/043801 (05.04.2012 Gazette 2012/14)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: 30.09.2010  JP 2010222998

(71) Applicant: Sekisui Medical Co., Ltd.
Tokyo 103-0027 (JP)

(72) Inventors:
• KOMODA Toshikazu
Amagasaki-shi
Hyogo 661-8564 (JP)
• NODA Yukihiko
Amagasaki-shi
Hyogo 661-8564 (JP)

(74) Representative: Ter Meer Steinmeister & Partner
Mauerkircherstraße 45
81679 München (DE)

(54) **PATCH**

(57) Provided is a patch that ensures high storage stability of donepezil or a salt thereof and allows stable transdermal administration of the donepezil or salt thereof in an amount that provides a pharmaceutical effect. The patch includes a substrate 1 and an adhesive layer 2 stacked on and integrated with one surface of the substrate 1 and containing donepezil or a salt thereof and an acrylic adhesive containing a copolymer obtained by copolymerizing monomers that each contain a (meth) acrylic acid ester of a saturated aliphatic alcohol and do not contain vinylpyrrolidone.

FIG.1

EP 2 623 103 A1

**Description**

Technical Field

The present invention relates to a patch that ensures high storage stability of donepezil or a salt thereof and allows stable transdermal administration of the donepezil or salt thereof.

Background Art

Recently, as the number of elderly persons increases, the number of Alzheimer's dementia patients is increasing, and this has become a social problem. As therapeutic agents for Alzheimer's disease, anticholinesterases such as donepezil hydrochloride have been developed and have been prescribed to the patients as internal medicines such as tablets.

However, as dementia progresses, the patient shows poor compliance, or swallowing dysfunction occurs. In such cases, it is often difficult to get the patient to take a therapeutic agent as directed by a physician. To solve this problem, there is a need for stable transdermal administration of the therapeutic agent.

Meanwhile, the transdermal permeability of a drug such as donepezil or a salt thereof is low, and therefore it is considered to be difficult to allow the drug to be absorbed through the skin in an amount that provides the desired pharmaceutical effect. To solve such a problem, various studies have been conducted, and the following transdermal absorption preparations containing donepezil or a salt thereof have been proposed.

Patent Literature 1 discloses a patch in which a combination of donepezil, a surfactant, and an acrylic adhesive is used to improve the transdermal permeability of donepezil. However, there is no description about the storage stability of donepezil.

Patent Literature 2 discloses suppression of discoloration of a donepezil-containing patch during storage, the donepezil-containing patch including an adhesive layer that is formed on one side of a substrate and contains donepezil and an acrylic adhesive with a specific water content. However, there is no description about the storage stability of donepezil.

Patent Literature 3 discloses a transdermal absorption preparation in which an acrylic adhesive layer containing donepezil and a metal chloride is cross-linked by a cross-linking agent to improve cohesion during removal by peeling. However, there is no description about the storage stability of donepezil.

Patent Literature 4 discloses a transdermal absorption-type dementia therapeutic preparation containing an adhesive composition, wherein the adhesive composition contains one or two or more kinds selected from the group consisting of donepezil and pharmaceutically acceptable salts thereof, and one or two or more kinds selected from the group consisting of organic acids and pharmaceutically acceptable salts thereof. However, there is no description about the storage stability of donepezil.

Patent Literature 5 discloses a patch having an adhesive layer on one side of a substrate, wherein the adhesive layer of the patch contains a fatty acid amide and boric acid. There is a description that the addition of the fatty acid amide and boric acid to the adhesive layer improves the transdermal permeability of donepezil and also improves the storage stability of donepezil. However, the storage stability of donepezil crystals is evaluated only after storage at 25°C for one month, and there is no description about the storage stability of the donepezil crystals at high temperatures that pharmaceuticals may temporarily experience during distribution thereof. When the patch is stored at high temperatures, the donepezil crystals may dissolve in the adhesive layer. Then when donepezil re-precipitates on the surface of the adhesive layer, the precipitation state of the donepezil crystals may become non-uniform, resulting in non-uniformity of the transdermal permeability of donepezil.

Citation List

Patent Literature

Patent Literature 1: WO2010/039381
Patent Literature 2: WO2009/145177
Patent Literature 3: WO2008/066179
Patent Literature 4: Japanese Patent No. 4394443
Patent Literature 5: Japanese Patent Application Laid-Open No. 2007-217328

Summary of Invention

Technical Problem

The present invention provides a patch that ensures high storage stability of donepezil or a salt thereof and allows stable transdermal administration of the donepezil or salt thereof in an amount that provides a pharmaceutical effect. Solution to Problem

The patch of the present invention comprises a substrate 1 and an adhesive layer 2 stacked on one side of the substrate 1 and integrated therewith, and is characterized in that the adhesive layer 2 contains donepezil or a salt thereof and an acrylic adhesive containing a copolymer obtained by copolymerizing monomers that each contain a (meth)acrylic acid ester of a saturated aliphatic alcohol and do not contain vinylpyrrolidone. Note that the (meth)acrylic acid means methacrylic acid or acrylic acid.

The adhesive layer 2 constituting the above-described patch contains donepezil or a salt thereof. Donepezil (2-[(1-benzyl-4-piperidinyl)methyl]-5,6-dimethoxyindan-1-one) or a salt thereof has an acetylcholinesterase inhibitory action and is used as an anti-Alzheimer's dementia drug.

Examples of the salt of donepezil may include: inorganic acid salts such as hydrochloride, sulfate, nitrate, hydrobromide, and phosphate; and organic acid salts such as acetate, trifluoroacetate, fumarate, maleate, lactate, tartrate, citrate, succinate, malonate, methanesulfonate, and p-toluenesulfonate. Of these, hydrochloride is preferred.

If the total amount of donepezil or a salt thereof contained in the adhesive layer 2 is low, a sufficient amount of the donepezil or salt thereof may not be released from the adhesive layer. If the total amount thereof is high, a large amount of crystals of the donepezil or salt thereof may precipitate on the surface of the adhesive layer, and this may cause the adhesion and flexibility of the adhesive layer to be reduced. In such a case, for example, the patch may peel off the skin during use, and stable transdermal administration of the donepezil or salt thereof may not be achieved. Therefore, the total amount of the donepezil or salt thereof is preferably 1 to 40% by weight, more preferably 5 to 35% by weight, and particularly preferably 10 to 30% by weight.

As described later, a reservoir layer 3 may intervene between the substrate 1 and the adhesive layer 2. In such a case, if the total amount of donepezil or a salt thereof contained in the adhesive layer 2 is low, a sufficient amount of the donepezil or salt thereof may not be released from the adhesive layer at the initial stage of administration. If the total amount thereof is high, a large amount of crystals of the donepezil or salt thereof may precipitate on the surface of the adhesive layer, and this may cause the adhesion and flexibility of the adhesive layer to be reduced. In such a case, for example, the patch may peel off the skin during use, and stable transdermal administration of the donepezil or salt thereof may not be achieved. Therefore, the total amount of the donepezil or salt thereof is preferably 0.5 to 30% by weight, more preferably 0.5 to 20% by weight, particularly preferably 1 to 15% by weight, and most preferably 1 to 5% by weight.

The above-described adhesive layer 2 contains the acrylic adhesive containing a copolymer obtained by copolymerizing monomers that each contain a (meth)acrylic acid ester of a saturated aliphatic alcohol and do not contain vinylpyrrolidone.

The copolymer contained in the acrylic adhesive is obtained by copolymerizing monomers that each contain a (meth)acrylic acid ester of a saturated aliphatic alcohol and do not contain vinylpyrrolidone. Note that the copolymer may be used alone, or two or more kinds of copolymers may be used in combination.

Examples of the saturated aliphatic alcohols may include monohydric saturated aliphatic alcohols ($R^1$-OH), dihydric saturated aliphatic alcohols, and trihydric saturated aliphatic alcohols. Of these, monohydric saturated aliphatic alcohols are preferred. A monohydric saturated aliphatic alcohol is obtained by substituting one hydrogen atom in an alkane with a hydroxyl group (-OH). A dihydric saturated aliphatic alcohol is obtained by substituting two hydrogen atoms in an alkane with hydroxyl groups (-OH). A trihydric saturated aliphatic alcohol is obtained by substituting three hydrogen atoms in an alkane with hydroxyl groups (-OH). Note that $R^1$ is an alkyl group.

The type of monohydric saturated aliphatic alcohol represented by $R^1$-OH to be used is not particularly limited, and examples thereof may include methyl alcohol, ethyl alcohol, n-propyl alcohol, isopropyl alcohol, n-butyl alcohol, hexyl alcohol, n-octyl alcohol, isooctyl alcohol, 2-ethylhexyl alcohol, decyl alcohol, dodecyl alcohol, tridecyl alcohol, and hexadecyl alcohol.

The type of dihydric saturated aliphatic alcohol to be used is not particularly limited, and examples thereof may include hydroxy ethyl alcohol and hydroxy propyl alcohol.

Examples of the trihydric saturated aliphatic alcohol may include glycerin.

In the monohydric saturated aliphatic alcohol represented by $R^1$-OH, if the number of carbon atoms in $R^1$ is small, the elasticity of the acrylic adhesive may become low, and the adhesion required for the patch may not be obtained. If the number of carbon atoms in $R^1$ is large, a liquid additive added to the acrylic adhesive to control its adhesion may exude from the adhesive layer over time, and the transdermal permeability and storage stability of donepezil or a salt thereof may change. Therefore, the number of carbon atoms in $R^1$ is preferably 2 to 16 and more preferably 3 to 16.

The (meth)acrylic acid ester of the saturated aliphatic alcohol is preferably a (meth)acrylic acid ester of a monohydric saturated aliphatic alcohol represented by $R^1$-OH. Examples of the (meth)acrylic acid ester of the saturated aliphatic alcohol may include methyl (meth)acrylate, ethyl (meth)acrylate, n-butyl (meth)acrylate, n-propyl (meth)acrylate, isopropyl (meth)acrylate, hexyl (meth)acrylate, n-octyl (meth)acrylate, isooctyl (meth)acrylate, 2-ethyhexyl (meth)acrylate, decyl (meth)acrylate, dodecyl (meth)acrylate, tridecyl (meth)acrylate, hexadecyl (meth)acrylate, hydroxyethyl acrylate, and hydroxypropyl acrylate. Of these, dodecyl (meth)acrylate and 2-ethylhexyl (meth)acrylate are preferred, and a combination of dodecyl (meth)acrylate and 2-ethylhexyl (meth)acrylate is more preferred. These (meth)acrylic acid esters of the saturated aliphatic alcohols may be used alone or two or more kinds of these may be used in combination. In the present invention, (meth)acrylate means methacrylate or acrylate.

The copolymer constituting the acrylic adhesive contains, as monomer components, at least two kinds of (meth)acrylic acid esters of saturated aliphatic alcohols. If the copolymer constituting the acrylic adhesive has pyrrolidone groups, the storage stability of donepezil or a salt thereof decreases. Therefore, the copolymer contains no vinylpyrrolidone as a monomer component.

The adhesion of the patch and its ability to dissolve and release donepezil or a salt thereof can be controlled by adjusting the kinds of (meth)acrylic acid esters of saturated aliphatic alcohols being the monomer components in the acrylic adhesive and the ratio of copolymerization. Therefore, the acrylic adhesive is suitable as an adhesive for supporting donepezil or a salt thereof.

The acrylic adhesive may further contain, as a monomer component, an additional monomer excepting vinylpyrrolidone. The addition of the additional monomer is preferred because the adhesion of the patch and its ability to dissolve and release donepezil or a salt thereof can be controlled with higher accuracy.

Examples of the additional monomer excepting vinylpyrrolidone may include itaconic acid, maleic acid, maleic anhydride, acrylamide, dimethylacrylamide, acrylonitrile, dimethylaminoethyl (meth)acrylate, t-butylaminoethyl (meth)acrylate, vinyl acetate, and vinyl propionate.

The acrylic adhesive may further contain a cross-linking agent as a monomer component. Examples of such a cross-linking agent may include polyisocyanate compounds, metal chelate compounds, metal alkoxide compounds, and epoxy compounds other than the above-described polyfunctional monomers. When the cross-linking agent is added as a monomer component of the acrylic adhesive, the internal cohesion of the acrylic adhesive is improved, and therefore the adhesive is less likely to remain on the skin when the patch is removed from the skin.

Any well-known method may be used as a polymerization method for producing the acrylic adhesive. Examples of the polymerization method may include a method in which the above-described monomers are mixed in the presence of a polymerization initiator to perform solution polymerization. More specifically, prescribed amounts of monomers, a polymerization initiator, and an optionally added cross-linking agent, together with a polymerization solvent, are supplied to a reaction vessel equipped with a stirrer and a reflux condenser for the vaporized solvent. Then, the mixture is heated at temperatures of 60 to 80°C for 4 to 48 hours to subject the monomers to a radical polymerization reaction.

Examples of the polymerization initiator may include: azobis-based polymerization initiators such as 2,2'-azobisisobutyronitrile (AIBN), 1,1'-azobis(cyclohexane-1-carbonitrile), and 2,2'-azobis-(2,4'-dimethylvaleronitrile); and peroxide-based polymerization initiators such as benzoyl peroxide (BPO), lauroyl peroxide (LPO), and di-tert-butyl peroxide. Examples of the polymerization solvent may include ethyl acetate and toluene. Preferably, the polymerization reaction is performed in a nitrogen gas atmosphere.

If the amount of the acrylic adhesive contained in the adhesive layer 2 is low, the cohesion of the adhesive layer becomes insufficient, and this may, for example, cause a problem in that the adhesive layer remains on the skin when the patch is removed from the skin. If the amount thereof is high, the adhesive layer may not be capable of containing donepezil or a salt thereof in an amount sufficient to obtain sufficient releasability of the donepezil or salt thereof from the adhesive layer. Therefore, the amount of the acrylic adhesive is preferably 40 to 99% by weight, more preferably 50 to 95% by weight, and particularly preferably 60 to 90% by weight.

As described later, a reservoir layer 3 may intervene between the substrate 1 and the adhesive layer 2. In such a case, if the total amount of the acrylic adhesive contained in the adhesive layer 2 is low, the cohesion of the adhesive layer becomes low, and this may cause a problem in that the adhesive layer remains on the skin when the patch is removed from the skin. If the total amount is high, the adhesive layer may not be capable of containing donepezil or a salt thereof in an amount sufficient to obtain sufficient releasability of the donepezil or salt thereof from the adhesive layer at the initial stage of administration. Therefore, the total amount of the acrylic adhesive is preferably 60 to 99.5% by weight, more preferably 70 to 99% by weight, and particularly preferably 75 to 98.5% by weight.

The adhesive layer 2 may further contain a resolvent for the purpose of improving the amount of donepezil or a salt thereof dissolved in the adhesive layer 2. Preferably, the adhesive layer 2 contains, as such a resolvent, at least one compound selected from the group consisting of esters of saturated aliphatic carboxylic acids with saturated aliphatic alcohols, saturated aliphatic alcohols, and saturated aliphatic carboxylic acids, because the transdermal permeability of donepezil or a salt thereof can be improved. By further improving the amount of the donepezil or salt thereof dissolved in the acrylic adhesive and improving the diffusibility of the donepezil or salt thereof in the acrylic adhesive, the excess of the donepezil or salt thereof that remains undissolved in the adhesive layer during production thereof can be rapidly precipitated on the surface of the adhesive layer. Therefore, more preferably, the adhesive layer 2 contains at least one compound selected from the group consisting of diesters of saturated aliphatic carboxylic acids with saturated aliphatic alcohols and triesters of saturated aliphatic carboxylic acids with saturated aliphatic alcohols.

If the total amount of the at least one compound selected from the group consisting of esters of saturated aliphatic carboxylic acids with saturated aliphatic alcohols, saturated aliphatic alcohols, and saturated aliphatic carboxylic acids contained in the adhesive layer 2 is low, the rate of precipitation of crystals of the excess of the donepezil or salt thereof that remains undissolved in the adhesive layer during production of the patch may become unacceptably low, and the precipitation state of the crystals of the donepezil or salt thereof on the surface of the adhesive layer may become non-uniform. If the total amount thereof is high, the resolvent may exude from the adhesive layer over time. Therefore, the total amount thereof is preferably 5 to 35% by weight and more preferably 10 to 30% by weight.

First, the esters of saturated aliphatic carboxylic acids with saturated aliphatic alcohols will be described. Examples of the esters of saturated aliphatic carboxylic acids with saturated aliphatic alcohols may include monoesters of saturated aliphatic carboxylic acids with saturated aliphatic alcohols, diesters of saturated aliphatic carboxylic acids with saturated aliphatic alcohols, and triesters of saturated aliphatic carboxylic acids with saturated aliphatic alcohols.

The saturated aliphatic alcohols are preferably monohydric saturated aliphatic alcohols ($R^2$-OH), dihydric saturated aliphatic alcohols, and trihydric saturated aliphatic alcohols. A monohydric saturated aliphatic alcohol is obtained by substituting one hydrogen atom in an alkane with a hydroxyl group (-OH). A dihydric saturated aliphatic alcohol is obtained by substituting two hydrogen atoms in an alkane with hydroxyl groups (-OH). A trihydric saturated aliphatic alcohol is obtained by substituting three hydrogen atoms in an alkane with hydroxyl groups (-OH). Note that $R^2$ is an alkyl group.

In the monohydric saturated aliphatic alcohol represented by $R^2$-OH, if the number of carbon atoms in $R^2$ is large, the compatibility between the acrylic adhesive and an ester of a saturated aliphatic carboxylic acid with a saturated aliphatic alcohol may become low. In such a case, the ester of the saturated aliphatic carboxylic acid with the saturated aliphatic alcohol may exude from the surface of the adhesive layer during production of the patch, and this may cause a reduction in the adhesion of the adhesive layer. Therefore, the number of carbon atoms in $R^2$ is preferably 1 to 22 and further preferably 1 to 18.

The type of monohydric saturated aliphatic alcohol to be used is not particularly limited, and examples thereof may include methyl alcohol, ethyl alcohol, n-butyl alcohol, n-propyl alcohol, isopropyl alcohol, hexyl alcohol, n-octyl alcohol, isooctyl alcohol, 2-ethylhexyl alcohol, decyl alcohol, dodecyl alcohol, tridecyl alcohol, tetradecyl alcohol (myristyl alcohol), hexadecyl alcohol, isostearyl alcohol, hexyldecanol, and octyldodecanol.

The type of dihydric saturated aliphatic alcohol to be used is not particularly limited, and examples thereof may include hydroxy ethyl alcohol and hydroxy propyl alcohol.

Examples of the trihydric saturated aliphatic alcohol may include glycerin.

The saturated aliphatic carboxylic acid is preferably any of saturated aliphatic monocarboxylic acids, saturated aliphatic dicarboxylic acids, and saturated aliphatic tricarboxylic acids.

A saturated aliphatic monocarboxylic acid is a carboxylic acid ($R^3$-COOH) obtained by substituting one hydrogen atom in an alkane with a carboxy group (-COOH). Note that $R^3$ is an alkyl group or a substituent in which one or two or more hydrogen atoms in an alkyl group are substituted with hydroxyl groups. The type of saturated aliphatic monocarboxylic acid to be used is not particularly limited, and examples thereof may include myristic acid, isostearic acid, and lauric acid.

In the carboxylic acid represented by $R^3$-COOH, if the number of carbon atoms in $R^3$ is large, the compatibility between the acrylic adhesive and an ester of a saturated aliphatic carboxylic acid with a saturated aliphatic alcohol may become low. In such a case, the ester of the saturated aliphatic carboxylic acid with the saturated aliphatic alcohol may exude from the surface of the adhesive layer during production of the patch, and this may cause a reduction in the adhesion of the adhesive layer. Therefore, the number of carbon atoms in $R^3$ is preferably 1 to 22 and more preferably 1 to 18.

A saturated aliphatic dicarboxylic acid is a carboxylic acid obtained by substituting two hydrogen atoms in an alkane with carboxy groups (-COOH), and one or two or more hydrogen atoms may be further substituted with hydroxyl groups. The type of saturated aliphatic dicarboxylic acid to be used is not particularly limited, and examples thereof may include sebacic acid and adipic acid.

A saturated aliphatic tricarboxylic acid is a carboxylic acid obtained by substituting three hydrogen atoms in an alkane with carboxy groups (-COOH), and one or two or more hydrogen atoms may be further substituted with hydroxyl groups. The type of saturated aliphatic tricarboxylic acid to be used is not particularly limited, and examples thereof may include citric acid.

Examples of the monoesters of saturated aliphatic carboxylic acids with saturated aliphatic alcohols may include monoesters of saturated aliphatic monocarboxylic acids with monohydric saturated aliphatic alcohols represented by $R^2$-OH. Examples of the monoesters of saturated aliphatic monocarboxylic acids with monohydric saturated aliphatic

alcohols represented by $R^2$-OH may include isopropyl myristate and hexyl laurate.

Examples of the diesters of saturated aliphatic carboxylic acids with saturated aliphatic alcohols may include: diesters of saturated aliphatic dicarboxylic acids with monohydric saturated aliphatic alcohols represented by $R^2$-OH; and diesters of saturated aliphatic monocarboxylic acids with dihydric saturated aliphatic alcohols obtained by substituting two hydrogen atoms in an alkane with hydroxyl groups (-OH). Of these, diesters of saturated aliphatic dicarboxylic acids with monohydric saturated aliphatic alcohols represented by $R^2$-OH are preferred. Examples of the diesters of saturated aliphatic carboxylic acids with saturated aliphatic alcohols may include diethyl sebacate, diisopropyl sebacate, and diisopropyl adipate.

Examples of the triesters of saturated aliphatic carboxylic acids with saturated aliphatic alcohols may include: triesters of saturated aliphatic tricarboxylic acids with monohydric saturated aliphatic alcohols represented by $R^2$-OH; and triesters of saturated aliphatic monocarboxylic acids with trihydric saturated aliphatic alcohols obtained by substituting three hydrogen atoms in an alkane with hydroxyl groups (-OH). Of these, triesters of saturated aliphatic tricarboxylic acids with monohydric saturated aliphatic alcohols represented by $R^2$-OH are preferred. Examples of the triesters of saturated aliphatic carboxylic acids with saturated aliphatic alcohols may include triethyl citrate and triacetin.

The saturated aliphatic alcohol used as the resolvent is preferably any of monohydric saturated aliphatic alcohols ($R^4$-OH), dihydric saturated aliphatic alcohols, and trihydric saturated aliphatic alcohols. A monohydric saturated aliphatic alcohol is obtained by substituting one hydrogen atom in an alkane with a hydroxyl group (-OH). A dihydric saturated aliphatic alcohol is obtained by substituting two hydrogen atoms in an alkane with hydroxyl groups (-OH). A trihydric saturated aliphatic alcohol is obtained by substituting three hydrogen atoms in an alkane with hydroxyl groups (-OH). Note that $R^4$ is an alkyl group.

In the monohydric saturated aliphatic alcohol represented by $R^4$-OH, if the number of carbon atoms in $R^4$ is large, the compatibility between the monohydric saturated aliphatic alcohol and the acrylic adhesive may become low. In such a case, the monohydric saturated aliphatic alcohol may exude from the surface of the adhesive layer, and this may cause a reduction in the adhesion of the adhesive layer. Therefore, the number of carbon atoms in $R^4$ is preferably 1 to 22 and more preferably 1 to 20.

The type of monohydric saturated aliphatic alcohol to be used is not particularly limited, and examples thereof may include methyl alcohol, ethyl alcohol, n-butyl alcohol, n-propyl alcohol, isopropyl alcohol, hexyl alcohol, n-octyl alcohol, isooctyl alcohol, 2-ethylhexyl alcohol, decyl alcohol, dodecyl alcohol, tridecyl alcohol, tetradecyl alcohol (myristyl alcohol), hexadecyl alcohol, isostearyl alcohol, hexyldecanol, octyldodecanol, and docosanol. Of these, octyldodecanol (octyldodecan-1-ol) is preferred. Of these, 2-octyldodecanol (2-octyldodecan-1-ol) is more preferred.

The type of dihydric saturated aliphatic alcohol to be used is not particularly limited, and examples thereof may include hydroxy ethyl alcohol and hydroxy propyl alcohol.

Examples of the trihydric saturated aliphatic alcohol may include glycerin.

The saturated aliphatic carboxylic acid used as the resolvent is preferably any of saturated aliphatic monocarboxylic acids, saturated aliphatic dicarboxylic acids, and saturated aliphatic tricarboxylic acids.

A saturated aliphatic monocarboxylic acid is a carboxylic acid ($R^5$-COOH) obtained by substituting one hydrogen atom in an alkane with a carboxy group (-COOH). Note that $R^5$ is an alkyl group or a substituent in which one or two or more hydrogen atoms in an alkyl group are substituted with hydroxyl groups. The type of saturated aliphatic monocarboxylic acid to be used is not particularly limited, and examples thereof may include myristic acid, isostearic acid, and lauric acid.

In the carboxylic acid represented by $R^5$-COOH, if the number of carbon atoms in $R^5$ is large, the compatibility between the carboxylic acid and the acrylic adhesive may become low. In such a case, the carboxylic acid may exude from the surface of the adhesive layer, and this may cause a reduction in the adhesion of the adhesive layer. Therefore, the number of carbon atoms in $R^5$ is preferably 1 to 22 and more preferably 1 to 18.

A saturated aliphatic dicarboxylic acid is a carboxylic acid obtained by substituting two hydrogen atoms in an alkane with carboxy groups (-COOH), and one or two or more hydrogen atoms may be further substituted with hydroxyl groups. The type of saturated aliphatic dicarboxylic acid to be used is not particularly limited, and examples thereof may include sebacic acid and adipic acid.

A saturated aliphatic tricarboxylic acid is a carboxylic acid obtained by substituting three hydrogen atoms in an alkane with carboxy groups (-COOH), and one or two or more hydrogen atoms may be further substituted with hydroxyl groups. The type of saturated aliphatic tricarboxylic acid to be used is not particularly limited, and examples thereof may include citric acid.

Preferably, the adhesive layer 2 contains, as resolvents, at least one compound selected from the group consisting of diesters of saturated aliphatic carboxylic acids with saturated aliphatic alcohols and triesters of saturated aliphatic carboxylic acids with saturated aliphatic alcohols and at least one compound selected from the group consisting of monoesters of saturated aliphatic monocarboxylic acids with monohydric saturated aliphatic alcohols and monohydric saturated aliphatic alcohols.

The diesters of saturated aliphatic carboxylic acids with saturated aliphatic alcohols and the triesters of saturated aliphatic carboxylic acids with saturated aliphatic alcohols are the same as those described above, and descriptions thereof will be omitted. The monoesters of saturated aliphatic monocarboxylic acids with monohydric saturated aliphatic alcohols are the same as those described above, and a description thereof will be omitted. The monohydric saturated aliphatic alcohols are the same as the monohydric saturated aliphatic alcohol represented by $R^4$-OH, and a description thereof will be omitted.

The solubility of donepezil or a salt thereof in the diesters of saturated aliphatic carboxylic acids with saturated aliphatic alcohols and the triesters of saturated aliphatic carboxylic acids with saturated aliphatic alcohols is greatly affected by temperature. When the patch is stored at high temperatures, part of crystals of the donepezil or salt thereof that are precipitated on the surface of the adhesive layer may dissolve in a diester of a saturated aliphatic carboxylic acid with a saturated aliphatic alcohol or a triester of a saturated aliphatic carboxylic acid with a saturated aliphatic alcohol, and therefore the state of precipitation of the donepezil or salt thereof on the surface of the adhesive layer may become non-uniform. This may cause non-uniformity of the transdermal permeability of the donepezil or salt thereof from the adhesive layer.

Therefore, at least one compound selected from the group consisting of monoesters of saturated aliphatic monocarboxylic acids with monohydric saturated aliphatic alcohols and monohydric saturated aliphatic alcohols, in which the solubility of donepezil or a salt thereof is not greatly affected by temperature, is combined with at least one compound selected from the group consisting of diesters of saturated aliphatic carboxylic acids with saturated aliphatic alcohols and triesters of saturated aliphatic carboxylic acids with saturated aliphatic alcohols. Such a combination can prevent non-uniformity of the state of precipitation of the donepezil or salt thereof on the surface of the adhesive layer even when the patch is stored at high temperatures.

In addition, the combination of at least one compound selected from the group consisting of diesters of saturated aliphatic carboxylic acids with saturated aliphatic alcohols and triesters of saturated aliphatic carboxylic acids with saturated aliphatic alcohols with at least one compound selected from the group consisting of monohydric saturated aliphatic alcohols and monoesters of saturated aliphatic monocarboxylic acids with monohydric saturated aliphatic alcohols allows the amount of donepezil or a salt thereof dissolved in the adhesive layer and the diffusibility of the donepezil or salt thereof in the adhesive layer to be appropriately controlled. The transdermal permeability of the donepezil or salt thereof can thereby be improved.

If the total amount of the at least one compound selected from the group consisting of diesters of saturated aliphatic carboxylic acids with saturated aliphatic alcohols and triesters of saturated aliphatic carboxylic acids with saturated aliphatic alcohols contained in the adhesive layer 2 is low, the rate of precipitation of crystals of the excess of donepezil or a salt thereof that remains undissolved in the adhesive layer during production of the patch may become unacceptably low, and the precipitation state of the crystals of the donepezil or salt thereof on the surface of the adhesive layer may become non-uniform. If the total amount thereof is high, the resolvent may exude from the adhesive layer over time, or the donepezil or salt thereof precipitated on the surface of the adhesive layer may dissolve in the resolvent when the patch is stored at high temperatures, so that, when crystals of the donepezil or salt thereof re-precipitate on the surface of the adhesive layer, the precipitation state of the crystals of the donepezil or salt thereof may become non-uniform. Therefore, the total amount thereof is preferably 5 to 25% by weight and more preferably 10 to 15% by weight.

If the total amount of the at least one compound selected from the group consisting of monoesters of saturated aliphatic monocarboxylic acids with monohydric saturated aliphatic alcohols and monohydric saturated aliphatic alcohols contained in the adhesive layer 2 is low, the rate of precipitation of crystals of the excess of donepezil or a salt thereof that remains undissolved in the adhesive layer during production of the patch may become unacceptably low, so that the precipitation state of the crystals of the donepezil or salt thereof may become non-uniform. Also, if the total amount thereof is low, the influence of temperature changes on the solubility of the donepezil or salt thereof in the adhesive layer may not be suppressed because of the at least one compound selected from the group consisting of diesters of saturated aliphatic carboxylic acids with saturated aliphatic alcohols and triesters of saturated aliphatic carboxylic acids with saturated aliphatic alcohols. If the total amount thereof is high, the resolvent may exude from the adhesive layer over time. Therefore the total amount thereof is preferably 3 to 30% by weight and more preferably 5 to 20% by weight.

The adhesive layer 2 may further contain a general-purpose resolvent such as liquid paraffin in addition to the above-described resolvents.

If the total amount of the resolvents contained in the adhesive layer 2 is low, the rate of precipitation of crystals of the excess of donepezil or a salt thereof that remains undissolved in the adhesive layer during production of the patch may become unacceptably low, and the precipitation state of the crystals of the donepezil or salt thereof on the surface of the adhesive layer may become non-uniform. If the total amount thereof is high, the cohesion of the adhesive layer may decrease, and this may, for example, cause a problem in that the adhesive layer remains on the skin when the patch is removed from the skin. Therefore, the total amount thereof is preferably 5 to 35% by weight and more preferably 10 to 30% by weight.

Note that the adhesive layer 2 may further contain additives such as a stabilizer and a filler, so long as the physical properties of the adhesive layer 2 are not impaired. However, preferably, the adhesive layer 2 contains no cellulose described later.

The stabilizer is added for the purpose of suppressing the oxidation and decomposition of donepezil or a salt thereof. Examples of the stabilizer may include antioxidants such as butylhydroxytoluene and tocopherol acetate, cyclodextrin, and ethylenediaminetetraacetic acid. Preferably, the stabilizer is contained in the adhesive layer 2 in an amount of 0.05 to 10% by weight.

The filler is added in order to control the adhesion of the patch and the transdermal absorbability of donepezil or a salt thereof. Examples of such a filler may include: inorganic fillers such as silicic acid anhydride, titanium oxide, and zinc oxide; organic metal salts such as calcium carbonate and magnesium stearate; lactose; cellulose derivatives such as crystalline cellulose, ethyl cellulose, and low substituted hydroxypropyl cellulose; polyacrylic acid; and polymethacrylic acid. Preferably, the filler is contained in the adhesive layer 2 in an amount of 1 to 15% by weight.

The patch may not include the reservoir layer described later. In such a case, if the thickness of the adhesive layer 2 is small, the adhesive layer may not be capable of containing donepezil or a salt thereof in an amount necessary to obtain the desired blood level of the drug. If the thickness is large, the adhesive layer may bulge out of the substrate during storage or use of the patch, and the adhesive feeling of the patch may deteriorate. Also, the time required to remove the solvent during the production of the adhesive layer may increase, and this causes a reduction in the production efficiency of the patch. Therefore, the thickness of the adhesive layer 2 is preferably 10 to 500 $\mu$m, more preferably 50 to 200 $\mu$m, and particularly preferably 80 to 150$\mu$ m.

The substrate 1 constituting the patch of the present invention is stacked on and integrated with the adhesive layer 2. The substrate 1 prevents loss of the drug in the adhesive layer 2 and protects the adhesive layer 2. In addition, the substrate 1 is required to have strength sufficient to provide self-supportability to the patch and also have flexibility sufficient to provide good adhesive feeling to the patch.

The type of such substrate 1 to be used is not particularly limited, and examples thereof may include a resin sheet, a foamed resin sheet, a nonwoven fabric, a woven fabric, a knitted fabric, and an aluminum sheet. The substrate 1 may be composed of a single layer or a plurality of stacked and integrated layers.

Examples of the resin forming the above resin sheet may include cellulose acetate, ethyl cellulose, rayon, polyethylene terephthalate, plasticized vinyl acetate-vinyl chloride copolymers, nylon, ethylene-vinyl acetate copolymers, plasticized polyvinyl chloride, polyurethane, polyethylene, polypropylene, and polyvinylidene chloride. Of these, polyethylene terephthalate is preferred.

Preferably, the substrate 1 is prepared by stacking and integrating a polyethylene terephthalate sheet and a nonwoven fabric or a soft resin sheet, from the viewpoint of the flexibility of the substrate and the effect of preventing loss of donepezil or a salt thereof. A substrate prepared by stacking and integrating a polyethylene terephthalate sheet and a nonwoven fabric is more preferred. Examples of the material forming the nonwoven fabric may include polyethylene, polypropylene, ethylene-vinyl acetate copolymers, ethylene-methyl (meth)acrylate copolymers, nylon, polyester, vinylon, SIS copolymers, SEBS copolymers, rayon, and cotton. Of these, polyester is preferred. Note that these materials may be used alone, or two or more kinds thereof may be used in combination.

Preferably, for the purpose of preventing loss of the drug in the adhesive layer 2 of the patch of the present invention and protecting the adhesive layer 2, release paper 5 is releasably stacked on and integrated with the surface of the adhesive layer 2 of the patch.

Examples of the release paper 5 may include paper and resin films formed of polyethylene terephthalate, polyethylene, polypropylene, polyvinyl chloride, or polyvinylidene chloride. Preferably, the surface of the release paper 5 that faces the adhesive layer 2 has been subjected to release treatment. Note that the above release paper 5 may be composed of a single layer or a plurality of layers.

For the purpose of improving the barrier properties of the release paper, aluminum foil or an aluminum layer formed by vapor deposition may be provided to the release paper. When the release paper is formed of paper, the release paper may be impregnated with a resin such as polyvinyl alcohol, for the purpose of improving the barrier properties of the release paper.

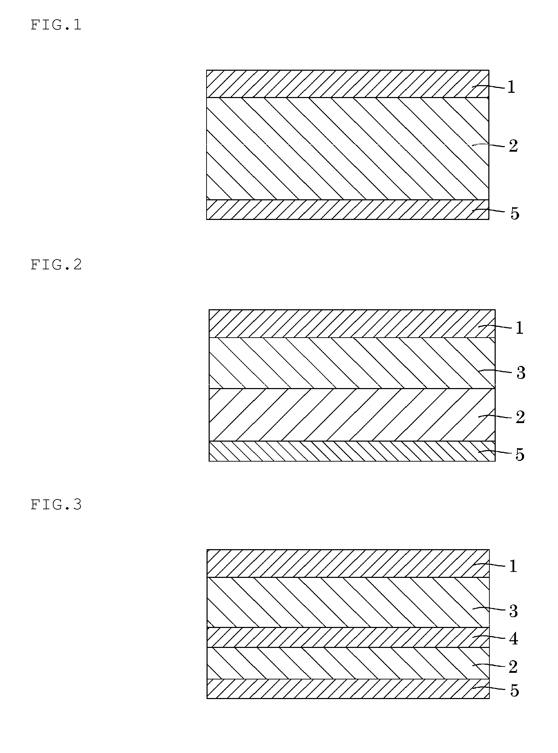

As shown in FIG. 2, a reservoir layer 3 may intervene between the substrate 1 and the adhesive layer 2 in order to improve the sustained release of donepezil or a salt thereof from the patch. The reservoir layer 3 contains cellulose and donepezil or a salt thereof. The type of cellulose contained in the reservoir layer 3 to be used is not particularly limited, and examples thereof may include hydroxypropyl cellulose, hypromellose, and ethyl cellulose. Of these, ethyl cellulose is preferred. For example, the cellulose used may be commercially available cellulose for use as a pharmaceutical additive. Examples of the commercially available cellulose for use as a pharmaceutical additive may include NISSO HPC (hydroxypropyl cellulose, manufactured by Nippon Soda Co., Ltd.), TC-5 and SB-4 (hypromellose, manufactured by Shin-Etsu Chemical Co., Ltd.), and ETHOCEL (registered trademark) (ethyl cellulose, manufactured by The Dow Chemical Company).

The viscosity of the cellulose is preferably 3 to 300 cps and more preferably 7 to 200 cps. If the viscosity of the cellulose is low, the flexibility of the reservoir layer becomes low, and cracks etc. occur during production. Therefore, donepezil or a salt thereof may not be uniformly contained over the reservoir layer, or the rate of release of the donepezil or salt thereof may become excessively high. If the viscosity of the cellulose is high, the diffusibility of the donepezil or salt thereof in the reservoir layer may be reduced significantly, and the rate of release of the donepezil or salt thereof from the patch may become low. By controlling the viscosity of the cellulose, the desired sustained release of the donepezil or salt thereof can be obtained. Note that the viscosity of the cellulose can be measured by a routine method using a B-type viscometer.

If the amount of the cellulose contained in the reservoir layer 3 is low, the thickness of the reservoir layer may become non-uniform. If the amount thereof is high, the reservoir layer may not be capable of containing donepezil or a salt in an amount sufficient to release the donepezil or salt thereof from the patch for several days. Therefore, the amount of the cellulose is preferably 15 to 70% by weight and more preferably 30 to 50% by weight.

If the total amount of donepezil or a salt thereof contained in the reservoir layer 3 is low, it may not be possible to release a sufficient amount of the donepezil or salt thereof from the adhesive layer for several days. If the total amount thereof is high, the amount of crystals of the donepezil or salt thereof precipitated on the surface of the reservoir layer that faces the adhesive layer may become high, and the conformity between the adhesive layer and the reservoir layer may deteriorate. In such a case, the reservoir layer may peel off the skin during the use of the patch, so that stable transdermal administration of the donepezil or salt thereof may not be achieved. Therefore, the total amount thereof is preferably 20 to 60% by weight and more preferably 30 to 50% by weight.

Note that the reservoir layer 3 may contain the above-described acrylic adhesive, the above-described resolvent, the above-described stabilizer, and the above-described filler, so long as the physical properties of the reservoir layer 3 are not impaired.

The above-described resolvent not only improves the solubility of donepezil or a salt thereof in the reservoir layer but also facilitates the precipitation of crystals of the donepezil or salt thereof on the surface of the reservoir layer that faces the adhesive layer. When the donepezil or salt thereof precipitates on the surface of the reservoir layer that faces the adhesive layer, the concentration of the donepezil or salt thereof present near the interface between the reservoir layer and the adhesive layer can be made high, and the supply of the donepezil or salt thereof to the adhesive layer can be facilitated. The resolvent that facilitates the precipitation of the donepezil or salt thereof on the surface of the reservoir layer that faces the adhesive layer is preferably a compound that improves the diffusibility of the donepezil or salt thereof in the cellulose. Examples of such a resolvent may include triacetin, triethyl citrate, diisopropyl adipate, and isopropyl myristate. Of these, triacetin and isopropyl myristate are preferred.

If the thickness of the reservoir layer 3 is small, the reservoir layer may not be capable of containing donepezil or a salt in an amount sufficient to release the donepezil or salt thereof from the patch for several days. If the thickness is large, the flexibility of the patch becomes low, and the patch may peel off the skin during use. Therefore, the thickness of the reservoir layer 3 is preferably 5 to 400 $\mu$m, more preferably 10 to 300 $\mu$m, and particularly preferably 20 to 150 $\mu$m.

In the case of the patch including the reservoir layer, if the thickness of the adhesive layer 2 is small, the adhesive layer may not be capable of containing donepezil or a salt thereof in an amount necessary to obtain the desired blood level of the drug at the initial stage of administration. If the thickness is large, the adhesive layer may bulge out of the substrate during storage or use of the patch, and the adhesive feeling of the patch may deteriorate. Also, the time required to remove the solvent during the production of the adhesive layer may increase, and this causes a reduction in the production efficiency of the patch. Therefore, the thickness of the adhesive layer 2 is preferably 10 to 200 $\mu$m, more preferably 20 to 150 $\mu$m, and particularly preferably 30 to 100 $\mu$m.

The adhesive layer 2 may be directly stacked on and integrated with the reservoir layer 3 as shown in FIG. 2. Alternatively, as shown in FIG. 3, an intermediate layer 4 such as a synthetic resin film, e.g., a polyolefin-based resin film such as a polyethylene film or a polypropylene film, may be interposed between the reservoir layer 3 and the adhesive layer 2, in order to control the rate of release of donepezil or a salt thereof from the reservoir layer to the adhesive layer. Preferably, a large number of through holes are formed in the synthetic resin film. The thickness of the intermediate layer 4 is preferably 1 $\mu$m or less.

A method of producing the patch of the present invention will next be described. The type of the method of producing the patch to be used is not particularly limited. Examples of the production method may include: (1) a method including adding donepezil or a salt thereof and an acrylic adhesive to a solvent such as ethyl acetate and optionally adding an additive thereto, stirring the mixture uniformly to obtain an adhesive layer solution, applying the obtained adhesive layer solution to one side of a substrate by a commonly used procedure, drying the adhesive layer solution to stack an adhesive layer on the one side of the substrate and integrate the adhesive layer with the substrate, and optionally stacking-integrating release paper on-with the adhesive layer such that the surface of the release paper that has been subjected to release treatment faces the adhesive layer: and (2) a method including applying the adhesive layer solution to the surface of release paper that has been subjected to release treatment by a commonly used coating method, drying the adhesive layer solution to form an adhesive layer on the release paper, and stacking-integrating a substrate on-with the adhesive layer.

When the reservoir layer 3 is interposed between the substrate 1 and the adhesive layer 2, the patch may be produced as follows. For example, first, the above adhesive layer solution is applied to the surface of release paper that has been subjected to release treatment by a commonly used coating procedure and then dried to form an adhesive layer on the release paper. Then a reservoir layer solution is prepared by adding cellulose, donepezil or a salt thereof, and optionally added additives to a solvent such as ethyl acetate and stirring the mixture until uniform. The reservoir layer solution is applied to the surface of separate release paper that has been subjected to release treatment by a commonly used coating procedure and then dried to form a reservoir layer on the release paper. Then the reservoir layer and the adhesive layer are stacked on and integrated with one surface of a substrate in that order. If necessary, release paper is stacked on and integrated with the adhesive layer such that the surface of the release paper that has been subjected to release treatment faces the adhesive layer, and a patch is thereby produced. When the intermediate layer 4 is interposed between the reservoir layer 3 and the adhesive layer 2, the reservoir layer 3, a film serving as the intermediate layer 4, and the adhesive layer 2 are stacked on and integrated with one surface of a substrate in that order to produce a patch. Advantageous Effects of Invention

The patch of the present invention has the above-described configuration. This ensures high storage stability of donepezil or a salt thereof and allows the donepezil or salt thereof to precipitate on the surface of the adhesive layer uniformly as crystals. Therefore, when the patch of the present invention is applied to the skin, the donepezil or salt thereof dissolved in the adhesive layer is transdermally administered. Then when the amount of the donepezil or salt thereof dissolved in the adhesive layer decreases, the crystals of the donepezil or salt thereof precipitated on the surface of the adhesive layer smoothly dissolve in the entire adhesive layer and are supplied thereto, and the donepezil or salt thereof is transdermally administered from the entire adhesive layer uniformly and stably.

Brief Description of Drawings

FIG. 1 is a vertical cross-sectional view showing a patch of the present invention.
FIG. 2 is a vertical cross-sectional view showing another example of the patch of the present invention.
FIG. 3 is a vertical cross-sectional view showing another example of the patch of the present invention.

Description of Embodiments

(Preparation of acrylic adhesive A)

An acrylic adhesive A was prepared as follows. A 40 L polymerization apparatus was charged with a reaction mixture composed of 50 parts by weight of ethyl acetate and monomers including 13 parts by weight of dodecyl methacrylate, 78 parts by weight of 2-ethylhexyl methacrylate, and 9 parts by weight of 2-ethylhexyl acrylate, and the atmosphere in the polymerization apparatus was replaced with a nitrogen atmosphere at 80°C. Then a polymerization initiator solution prepared by dissolving 0.5 parts by weight of benzoyl peroxide in 50 parts by weight of cyclohexane was added to the above reaction mixture over 24 hours to copolymerize the above monomers. After completion of the polymerization, ethyl acetate was further added to the resultant reaction mixture to obtain an acrylic adhesive solution A containing 35% by weight of the acrylic adhesive A.

(Adjustment of acrylic adhesive B)

A separable flask was charged with a reaction mixture composed of 200 parts by weight of ethyl acetate and monomers including 150 parts by weight of 2-ethylhexyl acrylate and 50 parts by weight of N-vinylpyrrolidone, and the atmosphere in the separable flask was replaced with a nitrogen atmosphere at 80°C. A polymerization initiator solution prepared by dissolving 1 part by weight of benzoyl peroxide in 100 parts by weight of ethyl acetate was added to the reaction mixture over 27 hours to polymerize the above monomers. After completion of the polymerization, ethyl acetate was further added to the resultant reaction mixture to obtain an acrylic adhesive solution B containing 32% by weight of an acrylic adhesive B.

(Examples 1 to 14)

Donepezil, the acrylic adhesive A, diisopropyl sebacate, diisopropyl adipate, isopropyl myristate, 2-octyldodecanol (2-octyldodecan-1-ol), and liquid paraffin were mixed in compositional weight ratios shown in TABLE 1. Then ethyl acetate was added to the mixtures such that the concentration of solids was 22% by weight, and the resultant mixtures were stirred until uniform to thereby produce adhesive layer solutions.

Next, 38 μm-thick polyethylene terephthalate films subjected to silicone release treatment were prepared, and the above-prepared adhesive layer solutions were applied to the silicone release-treated surfaces of the polyethylene terephthalate films and dried at 60°C for 30 minutes to thereby produce stacked bodies with adhesive layers with thicknesses shown in TABLE 1 formed on the silicone release-treated surfaces of the polyethylene terephthalate films.

Then 38 μm-thick polyethylene terephthalate films used as substrates were prepared and placed over the above-produced stacked bodies such that one sides of the substrates faced the adhesive layers of the stacked bodies. The adhesive layers of the stacked bodies were transferred to, stacked on, and integrated with the substrates, and patches were thereby produced.

TABLE 1

| | | EXAMPLE 1 | EXAMPLE 2 | EXAMPLE 3 | EXAMPLE 4 | EXAMPLE 5 | EXAMPLE 6 | EXAMPLE 7 | EXAMPLE 8 | EXAMPLE 9 | EXAMPLE 10 | EXAMPLE 11 | EXAMPLE 12 | EXAMPLE 13 | EXAMPLE 14 | COMPARATIVE EXAMPLE 1 |
|---|---|---|---|---|---|---|---|---|---|---|---|---|---|---|---|---|
| ADHESIVE LAYER | DONEPEZIL (% BY WEIGHT) | 4 | 12 | 12 | 12 | 12 | 12 | 12 | 20 | 20 | 24 | 12 | 12 | 25 | 10 | 4 |
| | ACRYLIC ADHESIVE A (% BY WEIGHT) | 96 | 88 | 63 | 63 | 63 | 63 | 58 | 60 | 60 | 51 | 58 | 58 | 63 | 80 | 0 |
| | ACRYLIC ADHESIVE B (% BY WEIGHT) | 0 | 0 | 0 | 0 | 0 | 0 | 0 | 0 | 0 | 0 | 0 | 0 | 0 | 0 | 96 |
| | DIISOPROPYL SEBACATE (% BY WEIGHT) | 0 | 0 | 25 | 0 | 15 | 15 | 0 | 0 | 0 | 15 | 0 | 0 | 0 | 0 | 0 |
| | DIISOPROPYL ADIPATE (% BY WEIGHT) | 0 | 0 | 0 | 25 | 0 | 0 | 15 | 10 | 10 | 0 | 20 | 15 | 8 | 0 | 0 |
| | ISOPROPYL MYRISTATE (% BY WEIGHT) | 0 | 0 | 0 | 0 | 0 | 10 | 15 | 10 | 0 | 10 | 10 | 15 | 4 | 0 | 0 |
| | 2-OCTYLDODECANOL (% BY WEIGHT) | 0 | 0 | 0 | 0 | 10 | 0 | 0 | 0 | 10 | 0 | 0 | 0 | 0 | 0 | 0 |
| | LIQUID PARAFFIN (% BY WEIGHT) | 0 | 0 | 0 | 0 | 0 | 0 | 0 | 0 | 0 | 0 | 0 | 0 | 0 | 10 | 0 |
| | THICKNESS (μm) | 100 | 100 | 100 | 100 | 100 | 100 | 100 | 100 | 100 | 100 | 100 | 100 | 100 | 100 | 100 |

(Examples 15 to 23)

Donepezil, ethyl cellulose (product name: "ETHOCEL Premium," manufactured by The Dow Chemical Company, viscosity: 7 cps), ethyl cellulose (product name: "ETHOCEL Premium," manufactured by The Dow Chemical Company, viscosity: 100 cps), triacetin, triethyl citrate, diisopropyl adipate, and isopropyl myristate were mixed in compositional weight ratios shown in TABLE 2. Then ethanol was added to the mixtures such that the concentration of solids was 25% by weight, and the resultant mixtures were stirred until uniform to thereby produce reservoir layer solutions.

Next, 38 μm-thick polyethylene terephthalate films subjected to silicone release treatment were prepared, and the above-prepared reservoir layer solutions were applied to the silicone release-treated surfaces of the polyethylene terephthalate films and dried at 80°C for 30 minutes to thereby produce first stacked bodies with reservoir layers with thicknesses shown in TABLE 2 formed on the silicone release-treated surfaces of the polyethylene terephthalate films.

Donepezil, the acrylic adhesive A, diisopropyl adipate, and isopropyl myristate were mixed in compositional weight ratios shown in TABLE 2. Then ethyl acetate was added to the mixtures such that the concentration of solids was 22% by weight, and the resultant mixtures were stirred until uniform to thereby produce adhesive layer solutions.

Separately, 38 μm-thick polyethylene terephthalate films subjected to silicone release treatment were prepared, and the above-prepared adhesive layer solutions were applied to the silicone release-treated surfaces of the polyethylene terephthalate films and dried at 60°C for 30 minutes to thereby produce second stacked bodies with adhesive layers with thicknesses shown in TABLE 2 formed on the silicone release-treated surfaces of the polyethylene terephthalate films.

Then 38 μm-thick polyethylene terephthalate films used as substrates were prepared and placed over the above-produced first stacked bodies such that one sides of the substrates faced the reservoir layers of the first stacked bodies. The reservoir layers of the first stacked bodies were transferred to, stacked on, and integrated with the substrates.

Next, these substrates were placed over the second stacked bodies such that the reservoir layers on the substrates faced the adhesive layers of the second stacked bodies. The adhesive layers of the second stacked bodies were transferred to, stacked on, and integrated with the reservoir layers, and patches were thereby produced.

TABLE 2

| | | EXAMPLE 15 | EXAMPLE 16 | EXAMPLE 17 | EXAMPLE 18 | EXAMPLE 19 | EXAMPLE 20 | EXAMPLE 21 | EXAMPLE 22 | EXAMPLE 23 | COMPARATIVE EXAMPLE 2 |
|---|---|---|---|---|---|---|---|---|---|---|---|
| RESERVOIR LAYER | DONEPEZIL (% BY WEIGHT) | 50 | 50 | 40 | 40 | 40 | 40 | 50 | 50 | 50 | 40 |
| | ETHYL CELLULOSE 7cps (% BY WEIGHT) | 50 | 50 | 40 | 40 | 40 | 40 | 0 | 0 | 0 | 40 |
| | ETHYL CELLULOSE 100cps (% BY WEIGHT) | 0 | 0 | 0 | 0 | 0 | 0 | 25 | 25 | 25 | 0 |
| | TRIACETIN (% BY WEIGHT) | 0 | 0 | 20 | 0 | 0 | 0 | 10 | 5 | 25 | 20 |
| | TRIETHYL CITRATE (% BY WEIGHT) | 0 | 0 | 0 | 20 | 0 | 0 | 0 | 0 | 0 | 0 |
| | DIISOPROPYL ADIPATE (% BY WEIGHT) | 0 | 0 | 0 | 0 | 20 | 0 | 0 | 0 | 0 | 0 |
| | ISOPROPYL MYRISTATE (% BY WEIGHT) | 0 | 0 | 0 | 0 | 0 | 20 | 15 | 20 | 0 | 0 |
| | THICKNESS ($\mu$m) | 80 | 60 | 50 | 50 | 50 | 50 | 70 | 70 | 70 | 50 |
| ADHESIVE LAYER | DONEPEZIL (% BY WEIGHT) | 1.5 | 1.5 | 1.5 | 1.5 | 1.5 | 1.5 | 1.5 | 1.5 | 1.5 | 1.5 |
| | ACRYLIC ADHESIVE A (% BY WEIGHT) | 98.5 | 78.5 | 78.5 | 78.5 | 78.5 | 78.5 | 78.5 | 78.5 | 78.5 | 0 |
| | ACRYLIC ADHESIVE B (% BY WEIGHT) | 0 | 0 | 0 | 0 | 0 | 0 | 0 | 0 | 0 | 78.5 |
| | DIISOPROPYL ADIPATE (% BY WEIGHT) | 0 | 10 | 10 | 10 | 10 | 10 | 10 | 10 | 10 | 10 |
| | ISOPROPYL MYRISTATE (% BY WEIGHT) | 0 | 10 | 10 | 10 | 10 | 10 | 10 | 10 | 10 | 10 |
| | THICKNESS ($\mu$m) | 40 | 40 | 40 | 40 | 40 | 40 | 40 | 40 | 40 | 40 |

(Comparative Example 1)

Donepezil and the acrylic adhesive B were mixed such that the mixture contains 4% by weight of the donepezil and 96% by weight of the acrylic adhesive B, and ethyl acetate was added such that the concentration of solids was 22% by weight. The resultant mixture was stirred until uniform to produce an adhesive layer solution. A patch including an adhesive layer having a thickness of 100 $\mu$m was produced as in Example 1 except that the above-produced adhesive layer solution was used.

(Comparative Example 2)

A patch including an adhesive layer having a thickness of 40 $\mu$m was produced as in Example 17 except that the acrylic adhesive B was used instead of the acrylic adhesive A.

The storage stability (in terms of content) and storage stability (in terms of precipitation state) of each of the patches obtained in the Examples and Comparative Examples were measured as described later, and the results are shown in TABLEs 3 and 4. In addition, each of the patches obtained in the Examples and Comparative Examples was subjected to a permeability test as described later to measure the cumulative skin permeation amount of donepezil. The results are shown in TABLEs 3 and 4. For each of the patches obtained in Examples 17, 21, and 23, the amount of transdermal delivery was measured as described later. The results are shown in TABLE 5.

(Storage stability (in terms of content))

Six test pieces with an area of 3 cm$^2$ were cut from a patch immediately after production and wrapped and sealed with a light-shielding wrapping material. Three of the test pieces were stored at 4°C for two weeks, and the other three were stored at 60°C for two weeks. After the storage, donepezil in each of the adhesive layers and reservoir layers was extracted with ethyl acetate, and the total amounts of donepezil in the adhesive layers and reservoir layers were quantified by HPLC. The total amount of donepezil in the adhesive layers and reservoir layers in the three test pieces stored at 4°C was denoted by $W_4$, and the total amount of donepezil in the adhesive layers and reservoir layers in the three test pieces stored at 60°C was denoted by $W_{60}$. The ratio of the remaining drug was computed using the following formula (1). The remaining ratio was entered in a "content" field in the "storage stability" row in TABLE 3.

$$\text{Remaining ratio (\% by weight)} = 100 \times W_{60}/W_4 \quad (1)$$

(Storage stability (in terms of precipitation state))

A square flat test piece with 3-cm side was cut from a patch immediately after production. The test piece was wrapped and sealed with a light-shielding wrapping material and stored at 25°C for one month. After one month, the test piece was observed from the adhesive layer side, and the precipitation state of crystals was observed under a transmission microscope at 40x.

A square flat test piece with 3-cm side was cut from the patch immediately after production. The test piece was wrapped and sealed with a light-shielding wrapping material and stored at 40°C for one month. After one month, the test piece was observed from the adhesive layer side, and the precipitation state of crystals was observed under a transmission microscope at 40x.

A square flat test piece with 3-cm side was cut from the patch immediately after production. The test piece was wrapped and sealed with a light-shielding wrapping material and stored at 60°C for one month. After one month, the test piece was observed from the adhesive layer side, and the precipitation state of crystals was observed under a transmission microscope at 40x.

When the crystals of donepezil were uniformly precipitated on the surface of the adhesive layer of a test piece, this test piece was evaluated as "good." When the crystals of donepezil were not uniformly precipitated, the test piece was evaluated as "bad." For each patch including the reservoir layer, the crystals of donepezil precipitated on the surface of the reservoir layer that faced the adhesive layer were projected in the direction of the thickness of the adhesive layer, and all the crystals of donepezil precipitated on the surface of the reservoir layer that faced the adhesive layer were considered to be precipitated on the surface of the adhesive layer.

(Permeability test)

A test piece with 1-cm side was cut from a patch immediately after production. Dorsal skin removed from a hairless mouse (male, 8 to 10 weeks old) was fixed on a Franz diffusion cell maintained at 37°C, and the test piece was applied to the upper end of the skin through the adhesive layer of the test piece. Note that a physiological saline solution with its pH adjusted to 7.2 was used as a receptor solution, and the lower end of the skin was immersed in the receptor solution.

The receptor solution on the lower side of the skin was collected 2, 4, 6, 22, and 24 hours after the test piece was applied to the skin, and the concentration of donepezil were measured by HPLC. More specifically, three test pieces were prepared, and the concentration of donepezil was measured for each test piece in the manner described above after 2, 4, 6, 22, and 24 hours. The permeation amount of donepezil determined from the donepezil concentration and the amount of the receptor solution was computed at each time point. The arithmetic mean of the permeation amounts of donepezil computed for the test pieces was determined at each time point, and the computed value was used as the cumulative skin permeation amount of donepezil. Note that when the permeation amounts of donepezil were computed after 4, 6, 22, and 24 hours, since the receptor solution had been collected before these time points, the permeation amounts were corrected on the basis of the amount of the receptor solution collected.

The permeation test was not performed for Comparative Examples 1 and 2 because the results for the storage stability (in terms of content) were lower than 90% by weight.

(Transdermal delivery amount)

Fifteen test pieces with an area of 3 $cm^2$ were cut from a patch immediately after production. Three test pieces were applied to each of four rabbits with shaved backs (male, 16 to 18 weeks old) through the adhesive layers of the test pieces. The rest of three test pieces were wrapped and sealed with a light-shielding wrapping material and stored at 4°C. One test piece was removed from the back skin one, four, and seven days after the application. Donepezil in the adhesive layer and reservoir layer of each test piece was extracted with ethyl acetate, and the total amount of donepezil in the adhesive layer and reservoir layer was quantified by HPLC (high performance liquid chromatography). The arithmetic mean of the total amounts of donepezil in test pieces was computed as a mean value $W_1$. For the three test pieces stored at 4°C and not applied to the back skin, one test piece was collected one, four, and seven days after the start of the measurement. Donepezil in the adhesive layer and reservoir layer of each test piece was extracted with ethyl acetate, and the total amount $W_0$ of donepezil in the adhesive layer and reservoir layer was quantified by HPLC (high performance liquid chromatography). The transdermal delivery amount of donepezil was computed using the following formula and entered in a "transdermal delivery amount" field in TABLE 5.

$$\text{Delivery amount (mg)} = W_0 - W_1$$

TABLE 3

| | | | EXAMPLE 1 | EXAMPLE 2 | EXAMPLE 3 | EXAMPLE 4 | EXAMPLE 5 | EXAMPLE 6 | EXAMPLE 7 | EXAMPLE 8 | EXAMPLE 9 | EXAMPLE 10 | EXAMPLE 11 | EXAMPLE 12 | EXAMPLE 13 | EXAMPLE 14 | COMPARATIVE EXAMPLE 1 |
|---|---|---|---|---|---|---|---|---|---|---|---|---|---|---|---|---|---|
| | CONTENT (% BY WEIGHT) | | 97.7 | 98.1 | 97.6 | 95.7 | 98.3 | 98.3 | 97.9 | 99.2 | 99.7 | 99.1 | 97.0 | 97.9 | 99.6 | 98.1 | 75.2 |
| STORAGE STABILITY | PRECIPITATION STATE | 25°C | GOOD | GOOD | GOOD | GOOD | GOOD | GOOD | GOOD | GOOD | GOOD | GOOD | GOOD | GOOD | GOOD | GOOD | BAD |
| | | 40°C | BAD | BAD | GOOD | GOOD | GOOD | GOOD | GOOD | GOOD | GOOD | GOOD | GOOD | GOOD | GOOD | BAD | BAD |
| | | 60°C | BAD | BAD | BAD | BAD | GOOD | GOOD | GOOD | GOOD | GOOD | GOOD | GOOD | GOOD | BAD | BAD | BAD |
| PERMEABILITY TEST | | 2 HOURS | 0.009 | 0.017 | 0.010 | 0.010 | 0.015 | 0.017 | 0.020 | 0.004 | 0.005 | 0.012 | 0.011 | 0.020 | 0.008 | 0.009 | — |
| | | 4 HOURS | 0.020 | 0.041 | 0.025 | 0.027 | 0.042 | 0.043 | 0.050 | 0.012 | 0.015 | 0.033 | 0.030 | 0.050 | 0.022 | 0.023 | — |
| | | 6 HOURS | 0.030 | 0.063 | 0.042 | 0.048 | 0.069 | 0.072 | 0.082 | 0.020 | 0.027 | 0.056 | 0.050 | 0.082 | 0.039 | 0.039 | — |
| | | 22 HOURS | 0.121 | 0.282 | 0.244 | 0.310 | 0.361 | 0.345 | 0.426 | 0.157 | 0.169 | 0.350 | 0.270 | 0.426 | 0.220 | 0.229 | — |
| | | 24 HOURS | 0.131 | 0.304 | 0.271 | 0.341 | 0.404 | 0.390 | 0.469 | 0.174 | 0.184 | 0.404 | 0.318 | 0.469 | 0.251 | 0.254 | — |

TABLE 4

| | | | EXAMPLE 15 | EXAMPLE 16 | EXAMPLE 17 | EXAMPLE 18 | EXAMPLE 19 | EXAMPLE 20 | EXAMPLE 21 | EXAMPLE 22 | EXAMPLE 23 | COMPARATIVE EXAMPLE 2 |
|---|---|---|---|---|---|---|---|---|---|---|---|---|
| | CONTENT (% BY WEIGHT) | | 98.1 | 97.3 | 98.5 | 96.9 | 97.8 | 97.4 | 98.8 | 98.4 | 98.5 | 80.4 |
| STORAGE STABILITY | PRECIPITATION STATE | 25°C | GOOD | GOOD | GOOD | GOOD | GOOD | GOOD | GOOD | GOOD | GOOD | GOOD |
| | | 40°C | BAD | GOOD | GOOD | GOOD | GOOD | GOOD | GOOD | GOOD | GOOD | GOOD |
| | | 60°C | BAD | GOOD | GOOD | GOOD | GOOD | GOOD | GOOD | GOOD | GOOD | GOOD |
| PERMEABILITY TEST | | 2 HOURS | 0.000 | 0.008 | 0.011 | 0.006 | 0.005 | 0.007 | 0.011 | 0.016 | 0.009 | — |
| | | 4 HOURS | 0.001 | 0.025 | 0.029 | 0.018 | 0.014 | 0.022 | 0.039 | 0.051 | 0.029 | — |
| | | 6 HOURS | 0.002 | 0.045 | 0.049 | 0.032 | 0.024 | 0.039 | 0.069 | 0.089 | 0.051 | — |
| | | 22 HOURS | 0.040 | 0.213 | 0.290 | 0.210 | 0.189 | 0.245 | 0.372 | 0.540 | 0.256 | — |
| | | 24 HOURS | 0.047 | 0.232 | 0.309 | 0.229 | 0.210 | 0.266 | 0.443 | 0.571 | 0.288 | — |

TABLE 5

| | | EXAMPLE 17 | EXAMPLE 21 | EXAMPLE 23 |
|---|---|---|---|---|
| TRANSDERMAL DELIVERY AMOUNT (mg) | ONE DAY | 0.50 | 0.46 | 0.38 |
| | FOUR DAYS | 1.00 | 0.95 | 0.62 |
| | SEVEN DAYS | 1.45 | 1.31 | 0.75 |

Industrial Applicability

The patch of the present invention ensures high storage stability of donepezil or a salt thereof and allows stable transdermal administration of the donepezil or salt thereof in an amount that provides a pharmaceutical effect. Therefore, the patch of the invention can be preferably used as a transdermal absorption preparation for Alzheimer's disease.
Reference Signs List

1    substrate
2    adhesive layer
3    reservoir layer

4     intermediate layer
5     release paper

**Claims**

1.  A patch comprising a substrate and an adhesive layer stacked on one side of the substrate and integrated therewith, the adhesive layer containing donepezil or a salt thereof and an acrylic adhesive containing a copolymer obtained by copolymerizing monomers that each contain a (meth)acrylic acid ester of a saturated aliphatic alcohol and do not contain vinylpyrrolidone.

2.  The patch according to claim 1, wherein the adhesive layer contains, as a resolvent, at least one compound selected from the group consisting of an ester of a saturated aliphatic carboxylic acid with a saturated aliphatic alcohol, a saturated aliphatic alcohol, and a saturated aliphatic carboxylic acid.

3.  The patch according to claim 2, wherein the adhesive layer contains, as the resolvent, at least one compound selected from the group consisting of a diester of a saturated aliphatic carboxylic acid with a saturated aliphatic alcohol and a triester of a saturated aliphatic carboxylic acid with a saturated aliphatic alcohol.

4.  The patch according to claim 3, wherein the diester of a saturated aliphatic carboxylic acid with a saturated aliphatic alcohol is a diester of a saturated aliphatic dicarboxylic acid with a saturated aliphatic alcohol.

5.  The patch according to claim 3, wherein the triester of a saturated aliphatic carboxylic acid with a saturated aliphatic alcohol is a triester of a saturated aliphatic tricarboxylic acid with a saturated aliphatic alcohol.

6.  The patch according to claim 2, wherein the adhesive layer contains, as the resolvents, at least one compound selected from the group consisting of a diester of a saturated aliphatic carboxylic acid with a saturated aliphatic alcohol and a triester of a saturated aliphatic carboxylic acid with a saturated aliphatic alcohol, and at least one compound selected from the group consisting of a monoester of a saturated aliphatic monocarboxylic acid with a monohydric saturated aliphatic alcohol and a monohydric saturated aliphatic alcohol.

7.  The patch according to any one of claims 1 to 6, comprising a reservoir layer between the substrate and the adhesive layer, with the reservoir layer containing cellulose and donepezil or a salt thereof

FIG.1

FIG.2

FIG.3

## INTERNATIONAL SEARCH REPORT

| | International application No. |
|---|---|
| | PCT/JP2011/072579 |

A. CLASSIFICATION OF SUBJECT MATTER
*A61K31/445*(2006.01)i, *A61K9/70*(2006.01)i, *A61K47/14*(2006.01)i, *A61K47/38* (2006.01)i, *A61P25/28*(2006.01)i

According to International Patent Classification (IPC) or to both national classification and IPC

B. FIELDS SEARCHED

Minimum documentation searched (classification system followed by classification symbols)
A61K31/445, A61K9/70, A61K47/14, A61K47/38, A61P25/28

Documentation searched other than minimum documentation to the extent that such documents are included in the fields searched
Jitsuyo Shinan Koho        1922–1996    Jitsuyo Shinan Toroku Koho    1996–2011
Kokai Jitsuyo Shinan Koho   1971–2011    Toroku Jitsuyo Shinan Koho    1994–2011

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y | WO 2007/129712 A1 (HISAMITSU PHARM CO., LTD., JP),<br>15 November 2007 (15.11.2007),<br>entire text; particularly, claims; examples 4 to 10<br>& EP 2016941 A1        & US 2009/175929 A1 | 1–6<br>7 |
| X<br>Y | WO 2008/044336 A1 (HISAMITSU PHARM CO., LTD., JP),<br>17 April 2008 (17.04.2008),<br>entire text; particularly, claims; example 5<br>(Family: none) | 1–6<br>7 |

[X] Further documents are listed in the continuation of Box C.    [ ] See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>07 November, 2011 (07.11.11) | Date of mailing of the international search report<br>15 November, 2011 (15.11.11) |
|---|---|
| Name and mailing address of the ISA/<br>Japanese Patent Office | Authorized officer |
| Facsimile No. | Telephone No. |

Form PCT/ISA/210 (second sheet) (July 2009)

**INTERNATIONAL SEARCH REPORT**

International application No.

PCT/JP2011/072579

C (Continuation).   DOCUMENTS CONSIDERED TO BE RELEVANT

| Category* | Citation of document, with indication, where appropriate, of the relevant passages | Relevant to claim No. |
|---|---|---|
| X<br>Y | WO 2006/082728 A1  (HISAMITSU PHARM CO., LTD., JP),<br>10 August 2006 (10.08.2006),<br>entire text; particularly, claims; examples 1 to 6<br>& US 2008/138388 A1 | 1-6<br>7 |
| X<br>Y | WO 2010/039381 A1  (TEIKOKU PHARMA USA INC., US),<br>08 April 2010 (08.04.2010),<br>entire text; particularly, claims; tables 1 to 3<br>& US 2010/080842 A1 | 1-6<br>7 |
| X<br>Y | WO 2008/066179 A1  (EISAI R & D MANAGEMENT CO., LTD., JP),<br>05 June 2008 (05.06.2008),<br>entire text; particularly, claims; examples 4, 8<br>& US 2008/131490 A1       & WO 2008/066183 A1<br>& WO 2008/066184 A1       & WO 2008/066185 A1<br>& WO 2008/066180 A1       & EP 2090310 A1<br>& EP 2098232 A1       & EP 2098233 A1<br>& EP 2098234 A1       & EP 2098235 A1<br>& US 2010/048628 A1 | 1-6<br>7 |
| X<br>Y | WO 2003/032960 A1  (HISAMITSU PHARM CO., LTD.),<br>24 April 2003 (24.04.2003),<br>entire text; particularly, claims; example 2<br>& EP 1437130 A1       & US 2004/258741 A1<br>& JP 4394443 B2 | 1-6<br>7 |
| X<br>Y | JP 2007-217328 A  (HISAMITSU PHARM CO., LTD.),<br>30 August 2007 (30.08.2007),<br>entire text; particularly, claims; examples 1 to 5<br>(Family: none) | 1-6<br>7 |
| Y | JP 2010-500992 A  (CORE TECH SOLUTIONS INC., US),<br>14 January 2010 (14.01.2010),<br>entire text; particularly, claims; fig. 1<br>& US 2008/044461 A1       & WO 2008/0021113 A2<br>& WO 2008/0021113 A3 | 1-7 |
| P,X<br>P,A | WO 2011/049038 A1  (TEIKOKU SEIYAKU CO., LTD., JP),<br>28 April 2011 (28.04.2011),<br>entire text; particularly, claims; example 22<br>(Family: none) | 1,2<br>3-7 |

Form PCT/ISA/210 (continuation of second sheet) (July 2009)

| | INTERNATIONAL SEARCH REPORT | International application No. |
|---|---|---|
| | | PCT/JP2011/072579 |

C (Continuation).   DOCUMENTS CONSIDERED TO BE RELEVANT

| Category* | Citation of document, with indication, where appropriate, of the relevant passages | Relevant to claim No. |
|---|---|---|
| P,X<br>P,A | WO 2011/074635 A1  (GOTO Takeshi),<br>23 June 2011 (23.06.2011),<br>entire text; particularly, claims; examples 1,<br>2<br>(Family: none) | 1-3,5,6<br>4,7 |
| P,X<br>P,A | WO 2011/074636 A1  (GOTO Takeshi),<br>23 June 2011 (23.06.2011),<br>entire text; particularly, claims; example 1<br>(Family: none) | 1-3,5<br>4,6,7 |

Form PCT/ISA/210 (continuation of second sheet) (July 2009)

**EP 2 623 103 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010039381 A
- WO 2009145177 A
- WO 2008066179 A
- JP 4394443 B
- JP 2007217328 A